# EUROPÄISCHE PATENTANMELDUNG

(11) **EP 1 369 118 A1**
(43) Veröffentlichungstag der Anmeldung: **10.12.2003**
(21) Anmeldenummer: 02012515.9
(22) Anmeldetag: 04.06.2002
(51) Int. Cl.: A61K 31/70

(54) **Verfahren und Mittel zur Prävention, Hemmung und Therapie von Sepsis**

(71) Anmelder: B.R.A.H.M.S Aktiengesellschaft, 16761 Hennigsdorf (DE)
(72) Erfinder: Bergmann, Andreas, Dr., 12351 Berlin (DE)
(74) Vertreter: Andrae, Steffen, Dr.

(57) **Zusammenfassung**

Verwendung der Ganglioside Asialo-G_{M1} (AG_{M1}) und/oder G_{M1} sowie von Substanzen, die den Kohlenhydratteil dieser Ganglioside im Hinblick auf eine Bindung an anti-AG_{M1}-Antikörper und/oder anti-G_{M1}-Antikörper simulieren, zur Herstellung eines Mittels zur Bindung oder Blockierung von anti-AG_{M1}-Antikörpern und/oder anti-G_{M1}-Antikörpern, die an natürliche Killerzellen (NKC) binden, für eine Verabreichung an Sepsisrisikopatienten und Sepsispatienten, bei denen derartige Antikörper nachgewiesen wurden, zur Prävention, Hemmung und Therapie von Sepsis.

## Beschreibung

Die vorliegende Erfindung betrifft neue Verfahren zur Sepsissprävention bzw. Sepsistherapie sowie die Mittel, die in derartigen Verfahren verwendbar sind. Sie beruht auf einem neuen diagnostischen Befund, nämlich dass in den Seren von Sepsispatienten mit hoher Sensitivität Antikörper (Autoantikörper) gefunden wurden, die die sogenannten "natürlichen Killerzellen" (NK-Zellen) inhibierende Eigenschaften aufweisen, sowie auf den präventiven und therapeutischen Maßnahmen, die die Anmelderin aus der kritischen Rolle abgleitet hat, die diese Antikörper aufgrund ihrer NK-Zellen inhibierenden Eigenschaften im Rahmen der Entwicklung einer Sepsis spielen können.

Insbesondere betrifft die vorliegende Erfindung Verfahren zur Prävention und Behandlung einer septischen Reaktion bei menschlichen Patienten (Sepsisrisikopatienten), bei denen sich im Nachgang zu einem medizinischen Eingriff und/oder einem Trauma (Unfall, Verbrennung, Kriegsverletzungen, Dekubitus u.ä.) eine Sepsis entwickelt hat oder noch entwickeln könnte, und, darüber hinaus gehend, auch zur Vermeidung einer potentiellen Gefährdung von Patienten durch eine septische Reaktion, die sich z.B. einem chirurgischen Eingriff unterziehen müssen, bei dem als gefährliche Komplikation eine Sepsis befürchtet werden muss, z.B. auf dem Feld der Viszeralchirurgie, der Transplantationsmedizin und der Hochdosischemotherapie in der Hämatologie/Onkologie.

Der Begriff "Sepsis" wird heute in einem engen Zusammenhang mit dem Begriff "Entzündung" verwendet. Als Entzündungen (Inflammationen) werden ganz allgemein bestimmte physiologische Reaktionen eines Organismus auf verschiedenartige äußere Einwirkungen wie z.B. Verletzungen, Verbrennungen, Allergene, Infektionen durch Mikroorganismen wie Bakterien, Pilze und Viren, auf Fremdgewebe, die Abstoßungsreaktionen auslösen, oder auf bestimmte entzündungsauslösende endogene Zustände des Körpers, z.B. bei Autoimmunerkrankungen und Krebs, bezeichnet. Entzündungen können als harmlose, lokal begrenzte Reaktionen des Körpers auftreten, sind jedoch auch typische Merkmale zahlreicher ernster chronischer und akuter Erkrankungen von einzelnen Geweben, Organen, Organ- und Gewebsteilen.

Lokale Entzündungen sind dabei meist Teil der gesunden Immunreaktion des Körpers auf schädliche Einwirkungen, und damit Teil des lebenserhaltenden Abwehrmechanismus des Organismus. Wenn Entzündungen jedoch Teil einer fehlgeleiteten Reaktion des Körpers auf bestimmte endogene Vorgänge wie z.B. bei Autoimmunerkrankungen sind und/oder chronischer Natur sind, oder wenn sie systemische Ausmaße erreichen, wie beim systemischen Inflammationssyndrom (Systemic Inflammatory Response Syndrome, SIRS) oder bei einer auf infektiöse Ursachen zurückzuführenden schweren Sepsis, geraten die für Entzündungsreaktionen typischen physiologischen Vorgänge außer Kontrolle und werden zum eigentlichen, häufig lebensbedrohlichen Krankheitsgeschehen. Zum modernen Sepsisbegriff kann beispielsweise verwiesen werden auf die Diskussion der Sepsis-Definition in K.Reinhart et al., "Sepsis und septischer Schock", in: Intensivmedizin, Georg Thieme Verlag, Stuttgart, New York, 2002, 756-760. Demgemäß "stellt das Krankheitsbild der Sepsis eine eigene pathophysiologische und klinische Entität dar, die auch unter Therapieaspekten von der zugrundeliegenden Infektion getrennt darzustellen und zu behandeln ist".

Es ist heute bekannt, dass die Entstehung und der Verlauf von entzündlichen Prozessen von einer Kaskade von Substanzen, die überwiegend proteinischer bzw. peptidischer Natur sind, gesteuert werden bzw. von einem mehr oder weniger zeitlich begrenzten Auftreten bestimmter Biomoleküle begleitet sind. Zu den an Entzündungsreaktionen beteiligten endogenen Substanzen gehören insbesondere solche, die zu den Cytokinen, Mediatoren, vasoaktiven Substanzen, Akutphasenproteinen und/oder hormonellen Regulatoren gezählt werden können. Die Entzündungsreaktion stellt somit eine komplexe physiologische Reaktion dar, an der sowohl das Entzündungsgeschehen aktivierende endogene Substanzen (z.B. TNF-α, Interleukin-1) als auch desaktivierende Substanzen (z.B. Interleukin-10) beteiligt sind.

Bei systemischen Entzündungen wie im Falle einer Sepsis bzw. des septischen Schocks weiten sich die entzündungsspezifischen Reaktionskaskaden unkontrolliert auf den gesamten Körper aus und werden dabei, im Sinne einer überschießenden oder entgleisenden Immunantwort, lebensbedrohlich.

Während wenigstens im europäischen Raum die durch eine positive Blutkultur nachweisbare systemische bakterielle Infektion lange den Sepsisbegriff prägte, wird die Sepsis heute in erster Linie als systemische Entzündung verstanden, die infektiöse Ursachen hat, als Krankheitsgeschehen jedoch große Ähnlichkeiten mit systemischen Entzündungen aufweist, die durch andere Ursachen ausgelöst werden. Der direkte Nachweis bakterieller Erreger wurde daher in jüngerer Zeit durch komplexe Überwachungen physiologischer Parameter und auch durch den Nachweis bestimmter nachweislich am Sepsisgeschehen bzw. am Entzündungsgeschehen beteiligter endogener Substanzen, d.h. spezifischer "Biomarker", ersetzt bzw. ergänzt.

Eine eingeführte, als Sepsis-Biomarker besonders geeignete endogene Substanz ist Procalcitonin. Die Bestimmung von Procalcitonin als Sepsismarker ist Gegenstand der Veröffentlichung M.Assicot, et al., "High serum procalcitonin concentrations in patients with sepsis and infection", The Lancet, vol.341, No.8844, 1993, 515-518; und der Patente DE 42 27 454 C2 bzw. EP 0 656 121 B1 bzw. US 5,639,617.

Die Verfügbarkeit des Sepsismarkers Procalcitonin hat der jüngeren Sepsisforschung starke Impulse gegeben, und es werden gegenwärtig intensive Anstrengungen unternommen, weitere Biomarker zu finden, die die Procalcitonin-Bestimmung ergänzen können und/oder zusätzliche Informationen für Zwecke der Feindiagnostik bzw. Differentialdiagnostik zu liefern vermögen. Ergebnisse dieser Anstrengungen finden sich in zahlreichen Patentanmeldungen der Anmelderin, und zwar in DE 198 47 690 A1 bzw. WO 00/22439 sowie in einer Reihe von noch unveröffentlichten deutschen Patentanmeldungen (DE 101 19 804.3 bzw. PCT/EP02/04219; DE 101 31 922.3; DE 101 30 985.6) bzw. europäischen Patentanmeldungen (EP 01128848.7; EP 01128849.5; EP 01128850.3; EP 01128851.1; EP 01128852.9; EP 01129121.8; EP 02008840.7; und EP 02008841.5). Auf den Inhalt der genannten Patente und Patentanmeldungen wird hiermit durch ausdrückliche Bezugnahme zur Ergänzung der vorliegenden Beschreibung verwiesen.

Da die bei Entzündungen gebildeten endogenen Substanzen Teil der komplexen Reaktionskaskade des Körpers sind, sind derartige Substanzen nicht nur von diagnostischem Interesse, sondern es wird gegenwärtig auch mit erheblichem Aufwand versucht, durch Beeinflussung der Entstehung und/oder der Konzentration einzelner derartiger Substanzen therapeutisch in das Entzündungsgeschehen einzugreifen, um die zum Beispiel bei Sepsis beobachtete systemische Ausbreitung der Entzündung möglichst frühzeitig zu stoppen. In diesem Sinne sind nachweislich am Entzündungsgeschehen beteiligte endogene Substanzen auch als potentielle therapeutische Targets anzusehen. Versuche, ansetzend an bestimmten Mediatoren des Entzündungsgeschehens dieses positiv therapeutisch zu beeinflussen, sind beschrieben beispielsweise in E.A.Panacek, "Anti-TNF strategies", Journal für Anästhesie und Intensivbehandlung; Nr.2, 2001, 4-5; T.Calandra, et al., "Protection from septic shock by neutralization of macrophage migration inhibitory factor", Nature Medicine, Vol.6, No.2, 2000, 164-170; oder K.Garber, "Protein C may be sepsis solution", Nature Biotechnology, Vol.18, 2000, 917-918. Angesichts der bisherigen eher enttäuschenden Ergebnisse derartiger therapeutischer Ansätze besteht ein starkes Interesse daran, weitere möglichst entzündungs- bzw. sepsisspezifische endogene Biomoleküle zu identifizieren, die auch als therapeutische Targets neue Erfolgsaussichten für die Sepsisprävention und -behandlung eröffnen.

Es kann somit als allgemeine Aufgabe der vorliegenden Erfindung bezeichnet werden, neue Maßnahmen (Verfahren, Mittel, Verwendungen) zur Sepsisprävention und -behandlung anzugeben, die sich aus dem Auffinden von bei Sepsis mit hoher Sensitivität auftretenden Biomolekülen ergeben, die eine zentraler Rolle bei der Entwicklung und im Verlauf einer Sepsis spielen.

Diese Aufgabe wird durch Verwendungen gemäß den Ansprüchen 1 bis 3 und deren Ausführungsformen gemäß den Ansprüchen 4 und 5 sowie Mittel gemäß Anspruch 6 gelöst.

Die vorliegende Erfindung beruht, generell gesprochen, auf dem überraschenden experimentellen Befund, dass ein bestimmter, an sich aus anderen Zusammenhängen bekannter Antikörper- bzw. Autoantikörper-Typ mit außerordentlich großer Häufigkeit in Seren von Patienten, die später eine Sepsis entwickelten oder bereits eine Sepsis hatten, mit hoher Sensitivität und signifikant erhöht gefunden wird, während der gleiche Antikörper bei gesunden Normalpersonen nicht oder nur in deutlich geringeren Mengen nachweisbar ist.

Dabei spielt für die in dieser Anmeldung beschriebenen präventiven und therapeutischen Maßnahmen eine entscheidende Rolle, dass die gefundenen Antikörper solche sind, die die Funktion der "natürlichen Killerzellen" (NK-Zellen; cytotoxische Lymphozyten) aufheben können, und dass es sich darüberhinaus erwiesen hat, dass verwandte, in anderen wissenschaftlichen Zusammenhängen bestimmte Antikörper mit diesen die NK-Zellen schädigenden Antikörpern kreuzreagieren.

Werden derartige, die NK-Zellen inhibierende Antikörper bei nahezu allen Sepsis-Patienten erhöht gefunden, werden sie daher zu einem therapeutischen Target zur Sepsisprävention und Sepsisbehandlung. Sie können, im Sinne der vorliegenden Erfindung, wie nachfolgend noch genauer erläutert wird, unter Anwendung von z.T. an sich grundsätzlich bekannten Maßnahmen bzw. erfindungsgemäßen Mitteln zur Bekämpfung, Bindung und Unschädlichmachung pathogener (Auto-)Antikörper und/oder von Maßnahmen mit dem Ziel einer gezielten Beeinflussung der Antikörperbildung durch das Immunsystem zum Ziel präventiver, hemmender und/oder therapeutischer Maßnahmen werden. Ferner kann ihr Nachweis im Vorfeld eines Sepsisrisiko-Ereignisses zur Veranlassung werden, erhöhte Sicherheitsmaßnahmen, z.B. im Sinne einer präventiven Antibiotika-Therapie, zur Vermeidung einer Sepsisentwicklung zu treffen.

Die vorliegende Erfindung geht, genauer gesagt, aus von den überraschenden Ergebnissen von Messungen der Anmelderin an Seren von Normalpersonen und Sepsispatienten mit Hilfe eines Ligandenbindungsassays mit erhöhter Sensitivität für an die Ganglioside AG_{M1} und G_{M1} bindende Antikörper. Das überraschende Ergebnis war, dass bei derartigen Messungen im wesentlichen in allen vermessenen Sepsisseren an Asialo-G_{M1} bindende Antikörper (anti-AG_{M1}-Antikörper) und/oder damit kreuzreagierende Antikörper, insbesondere an Monosialo-G_{M1} bindende Antikörper (anti-G_{M1}-Antikörpern) vom IgG- und/oder IgA-Typ gefunden wurden.

Die erhöhte Anwesenheit von Antikörpern, die an Asialo-G_{M1} binden und damit NK-Zellen inhibierende oder schädigende Eigenschaften aufweisen, in nahezu allen Seren von Sepsispatienten deutet darauf hin, dass derartige Antikörper an der Entwicklung einer Sepsis direkt beteiligt sind und/oder mit einer erhöhten Gefährdung eines Sepsisrisiokopatienten korrelierbar sind, d.h. mit der Gefahr, dass der Patient eine Sepsis entwickelt bzw. mit seiner individuellen Sepsis-Disposition.

Die physiologischen Bindungspartner der genannten Antikörper sind Ganglioside. Ganglioside sind Glykolipide, die Bestandteile der extrazellulären Seite der Plasmamembran tierischer Zellen sind und als solche auch im Nervengewebe auftreten. Sie enthalten pro Mol mehrere Monosaccharid-Einheiten, jedoch keinen Phosphor-Gehalt und werden den Sphingolipiden zugeordnet. Verglichen mit Proteinen sind sie eher niedermolekulare Biomoleküle. Die Ganglioside, an die die im Rahmen der vorliegenden Erfindung diskutierten Antikörper binden, sind das allgemein als G_{M1} bezeichnete Monosialo-Gangliosid bzw. insbesondere die zugehörige "Asialo"-verbindung AG_{M1}. G_{M1} weist eine Polysaccharidkette aus 4 Zucker-Monomereinheiten auf, die zwei D-Galactoseeinheiten, eine N-Acetylgalactosamin- und eine D-Glucoseeinheit umfassen, wobei letztere an einen sogenannten Ceramidteil gebunden ist. An die innerhalb der Polysaccharidkette angeordnete D-Galactoseeinheit ist bei dem Gangliosid G_{M1} ein N-Acetylneuraminsäurerest (NANA; Sialsäure- oder o-Sialinsäurerest; "Monosialo"-Rest) gebunden, der bei dem sialinsäurefreien Asialo-G_{M1} (AG_{M1}) fehlt.

Die genannten Ganglioside und verwandte Verbindungen werden mit zahlreichen wichtigen biologischen Funktionen des menschlichen Körpers in Zusammenhang gebracht, zu denen z.B. das axonale Wachstum und die neuronale Diffenzierung, Rezeptorenfunktionen und Beteiligungen an verschiedenen Immunreaktionen des Körpers sowie an der Signaltransduktion und Zell-Zell-Erkennung gehören.

Es ist seit langem bekannt, dass im menschlichen Körper in bestimmten Zusammenhängen Antikörper bzw. Autoantikörper nachgewiesen werden können, die an das Gangliosid G_{M1} binden, insbesondere dessen Kohlenhydratstrukturen, und an diesen ähnelnde ("diese simulierende") Kohlenhydratstrukturen anderer Moleküle. Die physiologische Rolle solcher Antikörper sowie ihre eventuelle Bedeutung für die klinische Diagnostik ist Gegenstand zahlreicher wissenschaftlicher Untersuchungen. Der Nachweis von anti-G_{M1}-Antikörpern wurde jedoch bisher nicht mit nachteiligen physiologischen Reaktionen korreliert, die eine Bedeutung für die Sepsisentwicklung aufweisen und die auf an sich bekannte Eigenschaften von an Asialo-G_{M1} bindenden Antikörpern zurückgeführt werden können.

Der weitaus überwiegende Teil aller veröffentlichten Arbeiten befaßt sich mit der Rolle und der diagnostischen Signifikanz von Anti-Gangliosid-Antikörpern bei Neuropathien, z.B. bei immunvermittelten motorischen Neuropathien wie dem Guillain-Barré-Syndrom (Radikuloneuritis, Polyradikulitis) und dem verwandten (Miller-)Fisher-Syndrom. Auch im Zusammenhang mit der Alzheimer-Erkrankung wurde bereits von einem vermehrten Auftreten von Anti-G_{M1}-Autoantikörpern bei einigen Patienten berichtet. Ferner wurden sie bei einzelnen HIV-Patienten gefunden. Dass sie auch bei Krebs mit einer sehr hohen Sensitivität auftreten, ist ein noch unveröffentlichter neuer Befund der Anmelderin, der den in den Europäischen Patentanmeldungen EP 02009884.4 und EP 02009882.8 offenbarten Erfindungen zugrunde liegt. Auf die beiden letztgenannten Europäischen Patentanmeldungen und die Literaturliste zu EP 02009884.4 wird hiermit zur Ergänzung der Ausführungen in der vorliegenden Anmeldung ebenfalls ausdrücklich verwiesen.

Nachfolgend werden die Befunde, die die Grundlage für die Erfindungen in der vorliegenden Anmeldung bilden, und die siche daraus ableitenden präventiven und therapeutischen Maßnahmen noch näher erläutert.

Zur Vermeidung von ungerechtfertigt engen und einschränkenden Auslegungen der in der vorliegenden Anmeldung und den zugehörigen Ansprüchen verwendeten Begriffe sollen nachfolgend einige der wichtigsten Begriffe für die Zwecke der vorliegenden Anmeldung besonders definiert werden:
- "Antikörper":: Dieser Begriff umfaßt, ohne zwischen verschiedenen Entstehungs- und Bildungsarten zu unterscheiden, Antikörper sowohl gegen externe Antigene als auch gegen körpereigene Strukturen, d.h. Autoantikörper, wobei letztere ggf. auch durch Antigen-Kreuzreaktionen aus Antikörpern gegen externe Antigene zu Autoantikörpern geworden sein können und ihre Bindungsfähigkeit gegen externe Antigene bewahrt haben können.
Wenn z.B. davon gesprochen wird, dass ein Antikörper "an Gangliosidstrukturen und an Gangliosidstrukturen simulierende Antigenstrukturen" bindet oder gegenüber "Gangliosiden bzw. bestimmten Gangliosiden reaktiv" ist, wobei reaktiv "im Sinne einer spezifischen Bindung reaktiv" bedeutet, soll er durch diese Definition hinreichend definiert sein, ohne dass z.B. seine spezifische Bindung auch an zusätzliche andere antigene Strukturen, oder seine praktische Bestimmung unter Verwendung von Reagenzien (zur Immobilisierung bzw. Markierung bzw. als Kompetitoren) mit molekularen Strukturen, die AG_{M1}, insbesondere dessen Kohlenhydratstuktur, nur simulieren, für die Definition als erfindungsgemäßer Antikörper eine Rolle spielen sollen.
- "Gangliosid": Im Rahmen der vorliegenden Erfindung steht der Begriff "Gangliosid" bei der Kennzeichnung des Bindungsverhaltens der zu bestimmenden Antikörper in erster Linie für das Ganglioside AG_{M1}. Der Begriff soll jedoch auch bisher noch nicht untersuchte verwandte Ganglioside erfassen, wenn sich noch zeigt, dass auch an diese Ganglioside bindende Antikörper mit einer vergleichbaren diagnostischen Signifikanz in Sepsisseren gefunden werden.
- "Simulation": Wenn im Rahmen der vorliegenden Anmeldung davon gesprochen wird, dass außer den speziellen Gangliosiden (AG_{M1} und G_{M1}) auch deren Bindungseigenschaften und Wirkungen "simulierende" Substanzen bzw. Verbindungen eingesetzt werden können, ist damit gemeint, dass es Kohlenhydratstrukturen aufweisende Verbindungen gibt (zu denen auch baterielle Toxine gehören), die wie die genannten Ganglioside an die fraglichen Antikörper binden. Deartige Verbindungen können daher potentiell wie die Ganglioside selbst zur spezifischen Bindung der Antikörper (z.B. zum Zweck ihrer Entfernung aus dem Blutkreislauf) oder zu ihrer Blockierung (und damit Unschädlichmachung) verwendet werden.
Im genannten Sinne das Bindungsverhalten von Gangliosiden "simulierende" Substanzen lassen sich z.B. mit Hilfe eines Screeningverfahrens auffinden, bei dem man eine biologische Probe (insbesondere ein Serum), die einen hohen anti-Gangliosid-Antikörper-Titer aufweist und deren Bindungsverhalten in einem gegebenen Assay bestimmt wurde, mit einer zu testenden Kandidaten-Substanz in Kontakt bringt und die Antikörperbindung in dem gleichen Assay ein weiteres Mal bestimmt. Eine deutliche Erniedrigung oder Aufhebung der Antikörperbindung im Assay ist ein Zeichen dafür, dass die untersuchte Substanz eine "Ganglioside simulierende Substanz" ist. Dieser Test kann auch im Zusammenhang mit der Festellung einer Patentverletzung durch Mittel, die den in der vorliegenden Anmeldung konkret offenbarten bzw. beanspruchten entsprechen, durchgeführt werden.

Weitere Begriffsbedeutungen ergeben sich für den Fachmann aus der einleitenden und nachfolgenden Beschreibung der Erfindung und ihrer Ausführungsbeispiele und der darin zitierten Literatur.

In der nachfolgenden Beschreibung wird zur Erläuterung der diagnostischen Befunde, die Grundlage der präventiven und therapeutisch nützlichen Lehren sind, auf Figuren Bezug genommen, die zeigen:
- Fig. 1: eine graphische Darstellung der Ergebnisse der Messung von Antikörpern der IgG-Klasse, die an Monosialo-G_{M1} binden, in Seren von 137 Kontrollpersonen, gegenübergestellt den Ergebnissen der Vermessung von 89 Seren von Sepsispatienten;
- Fig. 2: die Ergebnisse einer Vermessung der gleichen Seren wie in Fig. 1 auf Antikörper der IgA-Klasse, die an Monosialo-G_{M1} binden;
- Fig. 3: die Ergebnisse der Bestimmung von Antikörpern der IgG-Klasse, die an Asialo-G_{M1} binden, in Seren von 30 Normalpersonen (Kontrollen), gegenübergestellt den Ergebnissen der Vermessung von 20 Seren von Sepsispatienten (alle Seren sind Teilkollektive der in den Figuren 1 und 2 vermessenen Seren);
- Fig. 4: die Ergebnisse der Bestimmung von Antikörpern der IgA-Klasse, die an Asialo-G_{M1} binden, in den gleichen Seren wie in Fig. 3;

Die Erfindung beruht auf bei der Anmelderin durchgeführten Vermessungen von Seren, wobei in der vorliegenden Anmeldung im wesentlichen nur das Meßprinzip und die erhaltenen Ergebnisse wiedergegeben werden. Eine ausführlichere Offenbarung der durchgeführten Messungen findet sich in der älteren, unveröffentlichten Europäischen Patentanmeldung 02009884.4 der Anmelderin und einer gleichzeitig mit der vorliegenden Anmeldung eingereichten weiteren Anmeldung der Anmelderin. Auf beide Anmeldungen wird zur Ergänzung des Offenbarungsgehalts der vorliegenden Anmeldung ausdrücklich Bezug genommen:

### 1. Bestimmung von an die Ganglioside AG_{M1} und G_{M1} bindenden (Auto-)Antikörpern vom IgG- und IgA-Typ in Seren von gesunden Normalpersonen (Kontrollen) und Sepsispatienten

Unter Verwendung von mit Gangliosiden (AG_{M1} und G_{M1}) beschichteten Teströhrchen (GA-CTs), deren freie Bindungsstellen mit BSA abgesättigt worden waren, wurden Reihenmessungen an Kontroll- und Testseren vorgenommen. Dabei wurden, wie in der älteren, unveröffentlichten Anmeldung der Anmelderin EP 02009884.4 sowie einer gleichzeitig mit der vorliegenden Anmeldung eingereichten weiteren Europäischen Patentanmeldung der Anmelderin näher beschrieben ist, in einem ersten Inkubationsschritt an die zur Beschichtung verwendeten Ganglioside bindende Antikörper aus dem jeweiligen Serum (Kontroll- und Testserum) gebunden, und dann wurden zur separaten Bestimmung von Antikörpern vom IgG- und vom IgA-Typ die gebundenen Antikörper mit markierten tierischen (Ziege) anti human-IgG- bzw anti human-IgA-Immunglobulinen nachgewiesen. Die gebundenen markierten Immunglobuline wurden anhand ihrer Markierung (Acridiniumester als Chemilumineszenz-Marker) detektiert bzw. quantifiziert (in relativen Einheiten - "relativ units"; Bezugsbasis: Serum mit der höchsten Konzentration des jeweiligen zu bestimmenden Antikörpers).

Zur Gewinnung eines für die Antikörpermenge repräsentativen Messsignals muss dabei ein Hintergrundsignal in Abzug gebracht werden, das unter identischen Messbedingungen für das jeweilige identische Serum mit solchen Teströhrchen gewonnen wurde, die bis auf die fehlende Gangliosidbeschichtung mit den für die eigentlichen Messungen verwendeten Teströhrchen identisch waren.

Als Kontrollseren für die Antikörper-Bestimmungen unter Verwendung von GA-CTs, die mit G_{M1} beschichtet waren, dienten 137 Kontrollseren (Blutspenderseren und - zur Vermeidung von Lebensalter-bedingten Einflüssen auf die Antikörperkonzentrationen - Seren von Normalpersonen verschiedenen Alters aus Pflegeheimen, sowie von Mitarbeitern der Anmelderin). Für die Antikörper-Bestimmungen unter Verwendung von GA-CTs, die mit AG_{M1} beschichtet waren, wurde ein Teilkollektiv dieser Seren vermessen, das nur 30 Seren umfaßte.

Als Testseren für die Antikörper-Bestimmungen unter Verwendung von GA-CTs, die mit G_{M1} beschichtet waren, dienten 89 Seren von Sepsispatienten. Zu jedem Testserum existierte eine genaue klinische Dokumentation. Für die Antikörper-Bestimmungen unter Verwendung von GA-CTs, die mit AG_{M1} beschichtet waren, wurde ein Teilkollektiv dieser Seren vermessen, das nur 20 der Seren der Sepsispatienten umfaßte.

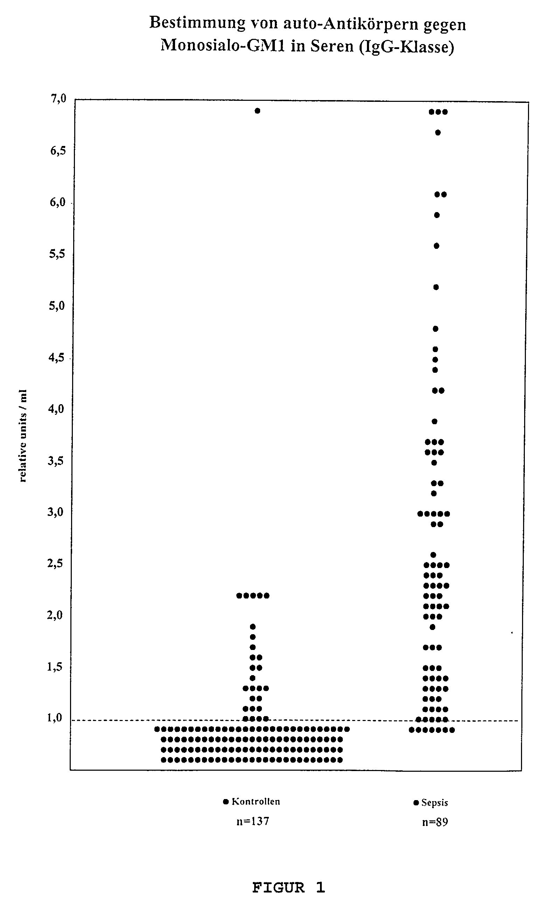

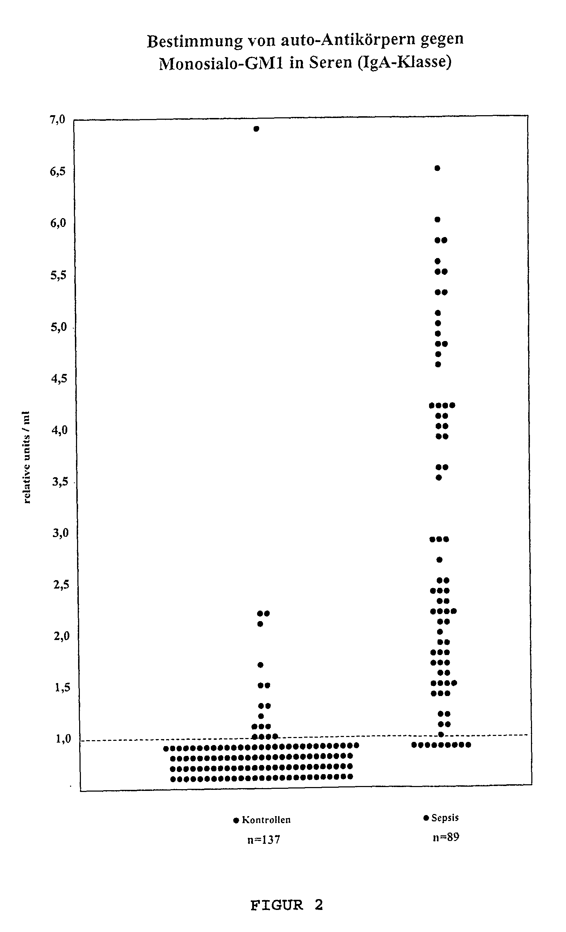

Die Ergebnisse der Bestimmungen von Antikörpern der Klassen IgG und IgA unter Verwendung von GA-CTs, die mit G_{M1} beschichtet waren, sind beispielhaft in den Figuren 1 und 2 gezeigt.

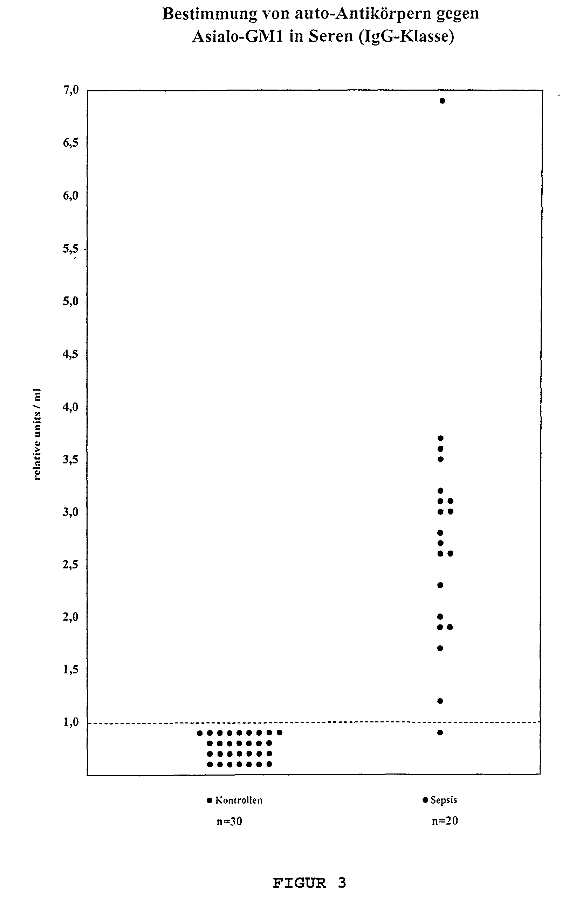

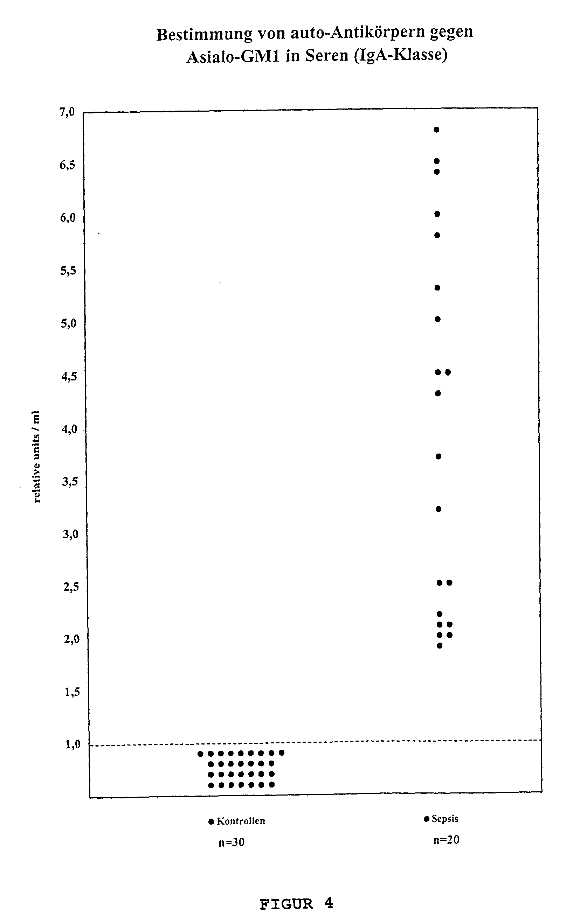

Die mit GA-CTs, die mit AG_{M1} beschichtet waren, erhaltenen entsprechenden Ergebnisse sind in den Figuren 3 und 4 gezeigt.

Es ist dabei ausdrücklich darauf hinzuweisen, dass zahlreiche Seren, in denen signifikant erhöhte anti-AG_{M1}- bzw. anti-G_{M1}-Antikörpertiter gefunden wurden, von Patienten stammten, denen die Blutprobe kurz (ca. 2h) nach dem Sepsisrisikoereignis entnommen worden war und die erst im späteren Verlauf die typischen Sepsissymptome entwickelten.

Es ist ferner darauf hinzuweisen, dass bei einem Versuch, analog zu den Bestimmungen der Antikörper vom IgG- und IgA-Typ auch die entsprechenden Antikörper vom IgM-Typ zu bestimmen, in den Sepsisseren keine diagnostisch relevant erhöhten Spiegel für solche Antikörper vom IgM-Typ gefunden wurden (Ergebnisse nicht gezeigt).

### 2. Diskussion der Befunde und erfindungsgemäße Maßnahmen zur Sepsisprävention und Therapie

Wie die in den Figuren 1 bis 4 zusammengefassten Messergebnisse eindrücklich zeigen, ermöglicht die Bestimmung von Antikörpern der Klassen IgA und/oder IgG, die an Ganglioside (AG_{M1} und/oder G_{M1}) binden, ein klare Unterscheidung der Kontrollgruppe von den Sepsispatienten, indem in nahezu allen (Fig.1: 82 von 89, d.h. 92%; Fig.2: 80 von 89, 90%; Fig.3: 19 von 20, 95%; Fig.4: alle 20, 100%) der untersuchten Sepsisseren deutlich erhöhte AG_{M1}- bzw. G_{M1}-Antikörpertiter gefunden werden.

Der Nachweis von deutlich erhöhten Konzentrationen von Antikörpern vom IgA und IgG-Typ auch in Patienten-Serumproben, die nur kurze Zeit (etwa 2h) nach dem "Sepsisrisiko-Ereignis" (z.B. Operation, Unfall, Verbrennung) gewonnen worden waren, sowie der mangelnde Nachweis von Antikörpern vom IgM-Typ, schließen es aus, dass die nachgewiesenen Antikörper erst infolge des "Sepsisrisiko-Ereignisses" bzw. einer damit verbundenen bakteriellen Infektion gebildet wurden. Das bedeutet aber, dass die Antikörper bei dem jeweiligen Sepsispatienten entweder schon vorher vorhanden waren und/oder dass die durch das Sepsis-Risikoereignis ausgelöst Aktivierung des vorsensibilisierten Immunsystems des Patienten in der Art eines "Booster"-Effekts zu einer intensiven Antikörperproduktion führte.

Die Tatsache, dass die AG_{M1}- bzw. G_{M1}-Antikörper in im wesentlichen allen vermessenen Sepsisseren (90 bis 100%) deutlich erhöht gefunden wurden, ist daher so zu interpretieren, dass die Ausbildung einer Sepsis entweder direkt auf das Vorhandensein der fraglichen Antikörper bei dem jeweiligen Patienten zurück zu führen ist oder wenigstens die Folge einer Aktivierung der aufgrund einer vorhergehenden Immunisierung bei dem Patienten bereits vorhandenen "molekularen Maschinerie" (in Form von B-Zellen) ist, die unter dem Einfluß des "Sepsisrisiko-Ereignisses" bzw. einer damit verbundenen Infektion ihre intensive Antikörperproduktion aufnimmt. Patienten ohne diese Antikörper bzw. die zu ihrer raschen Erzeugung erforderlichen Vorsensibilisierung entwickeln nach Maßgabe der bisherigen experimentellen Befunde eine Sepsis wahrscheinlich gar nicht oder nur erschwert.

Die beobachteten Befunde können erklärt werden: Es ist bekannt, dass die sogenannten "natürlichen Killerzellen" (NK-Zellen; cytotoxisch aktive Lymphozyten) an ihrer Oberfläche Asialo-G_{M1}-Strukturen aufweisen, an die anti-AG_{M1}-Antikörper spezifisch binden können und dadurch die NK-Zellen desaktivieren und zerstören. Das wird sogar planmäßig genutzt, indem auf dem Gebiet von Tierexperimenten, bei denen mit Versuchstieren gearbeitet wird, bei denen künstlich Tumoren erzeugt werden sollen, so gearbeitet wird, dass durch Gabe von anti-AG_{M1}-Antikörpern in Verbindung mit einem krebsauslösenden Karzinogen oder einem Tumorkeim die Immunabwehr des Versuchstiers ausgeschaltet wird, so dass sich der - im Tiermodell gewünschte - experimentelle Krebs entwickeln kann (Hugh F. Pross et al., Role of Natural Killer Cells in Cancer, Nat Immun 1993; 12:279-292; Lewis L. Lanier et al., Arousal and inhibition of human NK Cells, Immunological Reviews 1997, Vol. 155:145-154; Yoichi Fuji et al., IgG Antibodies to AsialoGM1 Are More Sensitive than IgM Antibodies to Kill *in vivo* Natural Killer Cells and Prematured Cytotoxic T Lymphocytes of Mouse Spleen, Microbiol.Immunol. Vol. 34(6), 533-542, 1990; N.Saijo et al., Analysis of Metastatic Spread and Growth of Tumor Cells in Mice with Depressed Natural Killer Activity by Anti-asialo GM1 Antibody or Anticancer Agents, J Cancer Res Clin Oncol (1984) 107: 157-163; Sonoku HABU et al., Role of Natural Kiler Cells against Tumor growth in Nude Mice - A Brief Review, Tokai J Exp Clin Med., Vol.8, No.5, 6: 465-468, 1983; Lewis L. Lanier, NK Cell Receptors, Annu. Rev. Immunol. 1998, 16: 359-93; Theresa L. Whiteside et al., The role of natural killer cells in immune surveillance of cancer; Current Opinion in Immunology 1995, 7:704-710; Tuomo Timonen et al., Natural killer cell-target cell interactions, Current Opinion in Cell Biology 1997, 9:667-673).

Aktive NK-Zellen spielen jedoch eine außerordentlich wichtige Rolle im Rahmen der Immunabwehr des Menschen, auch bei Sepsis bzw. schweren bakteriellen Infektionen. So beschreiben z.B. Shuiui Seki et al., in: Role of Liver NK Cells and Peritoneal Macrophages in Gamma Interferon and Interleukin-10 Production in Experimental Bacterial Peritonitis in Mice, Infection and Immunity, Vol. 66, No. 11, 1998, 5286-5294; die wichtige Rolle von NK-Zellen für die Produktion von entzündungsfördernden und entzündungshemmenden Cytokinen. Sie zeigen, dass eine künstliche Ausschaltung der NK-Zellen unter experimentellem Einsatz von anti-AG_{M1}-Antikörpern zu einer Inhibierung der Produktion des entzündungshemmenden Interferon-γ führt. Auch die Auswirkungen von chirurgischem Stress und einer Endotoxin-induzierten Sepsis auf die NK-Zell-Aktivität wurden bereits untersucht, und zwar in: P.Toft et al., in: The effect of surgical stress and endotoxin-induced sepsis on the NK-cell activity, distribution and pulmonary clearance of YAC-1 and melanoma cells, APMIS 1999; 107:359-364. Ein möglicher Einfluss von physiologisch gebildeten Antikörpern mit NK-Zell-Reaktivität wird jedoch in keiner der genannten Arbeiten berücksichtigt.

Der wissenschaftlichen Literatur lassen sich Erkenntnisse, die das erfindungsgemäße Verfahren nahelegen könnten, nicht entnehmen. In einigen Arbeiten wurde zwar eine Bestimmung von anti-Gangliosid-Antikörpern im Zusammenhang mit schweren akuten Infektionskrankheiten beschrieben. Eine solche Infektionskrankheit ist die durch den Parasiten *Trypanosoma cruzi* hervorgerufene Chagas-Krankheit (vgl. D.H.Bronia et al., in: Ganglioside treatment of acute *Trypanosoma cruzi* infection in mice promotes long-term survival and parasitological cure, Annals of Tropical Medicine & Parasitology, Vol. 93, No.4, 341-350 (1999) und die darin zitierte Literatur). In der letztgenannten Arbeit wird spekuliert, dass eine beobachtete, deutlich vorteilhafte Wirkung einer Verabreichung von exogenen Gangliosiden an mit dem Parasiten *T. cruzi* infizierte Mäuse bei diesen die Produktion von anti-Gangliosid-Antikörpern auslösen könnte, die dann mit Glycolipiden der Hülle von *T. cruzi* reagieren und dadurch den Tod des Parasiten bewirken sollen. Aufgrund der Befunde in der vorliegenden Anmeldung ist eine solche Erklärung wenig wahrscheinlich: Die im Falle der Chagas-Krankheit beobachteten anti-Gangliosid-Antikörper sind aufgrund ihrer die NK-Zellen hemmenden Wirkung nicht, wie angenommen, ein heilungsfördernder Faktor, sondern stattdessen ein krankheitsauslösender bzw. -fördernder Faktor. Durch die Verabreichung von exogenen Gangliosiden, die als solche nicht als Antigene angsehen werden können, werden die anti-AG_{M1}-Antikörper nämlich nicht gebildet sondern wahrscheinlich blockiert. Dadurch kann bei den Mäusen (bzw. bei Patienten) die Wirkung der NK-Zellen wiederhergestellt werden, und das Immunsystem kann der Parasiten Herr werden. Alle diesseits bekannten Arbeiten stellen keinerlei Zusammenhang zwischen anti-Asialo-G_{M1}-Antikörpern und der Entstehung und Verschärfung einer Sepsis her und können daher das erfindungsgemäße Verfahren weder vorwegnehmen noch nahelegen.

Das gilt auch für andere Arbeiten, die zeigen, dass eine Verabreichung von exogenen Gangliosiden bzw. bestimmten Gangliosidderivaten in Versuchtiermodellen entzündungshemmend wirkt bzw. die Überlebensrate von Mäusen, denen das Bakterientoxins LPS verabreicht worden war, signifikant erhöht (vgl. Silvia G. Correa et al., in: Anti-inflammatory effect of gangliosides in the rat hindpaw edema test, European Journal of Pharmacology, 199 (1991) 93-98; Amico-Roxas M., et al., in: Anti-inflammatory action of AGF₄₄, a ganglioside ester derivative, Drugs Exptl.Clin.Res.XVIII(6) 251-259 (1992); James J. Mond et al., in: Inhibition of LPS-Mediated Cell Activation In Vitro and In Vivo by Gangliosides, Ciruclatory Shock44:57-62 (1995). In keiner der Arbeiten werden die beobachteten Effekte mit Wechselwirkungen der verabreichten Ganglioside mit anti-Gangliosid-Antikörpern erklärt. Letztere werden im gegebenen Zusammenhang gar nicht diskutiert.

Dabei kann eine solche Wechselwirkung im Sinne einer Antikörperblockierung die beschriebenen Befunde schlüssig erklären. Die konzeptionellen Grundlagen der vorliegenden Erfindung lassen sich noch wie folgt vertiefen: Es ist davon auszugehen, dass die Sepsispatienten, deren Seren vermessen wurden, die bestimmten Antikörper bzw. die Anlage zu ihrer raschen Produktion (i.S. einer Sensibilisierung) bereits vor dem "Sepsisrisikoereignis" aufwiesen. Die Sensibilisierung eines Patienten im Hinblick auf die Produktion von anti-Gangliosid-Antikörpern kann dabei z.B. als Reaktion auf irgendeinen exogenen, antigenen Reiz (z.B. eine Allerweltsinfektion mit *Campylobacter jejuni* oder auch *Helicobacter pylori,* oder ggf. auch entsprechende Umweltsubstanzen) unabhängig von der späteren Sepsis erfolgt sein. Sie kann danach latent lange bestehen bleiben. Ist jedoch bei einem menschlichen Individuum die Produktion von anti-Asialo-G_{M1}-Antikörpern einmal initiiert bzw. stark erhöht, sind bei diesem Patienten, als Disposition, die Voraussetzungen gegeben, dass in bestimmten physiologischen Stress-Situationen wie Infektionen und anderen Ereignissen mit hoher NK-Zell-Aktivität (z.B. Zellentartung durch mutagene Ereignisse; eine Sepsis-Risikosituation) durch die anti-AG_{M1}-Antikörper und damit kreuzreagierenden Antikörper eine Schädigung der NK-Zellen und damit der Immunabwehr erfolgt. Es besteht ein erhöhtes Risiko, dass eine Abwehrreaktion, die durch z.B. einen "Sepsisrisiko-Stress" ausgelöst wird und ein Eingreifen der NK-Zellen erfordert, auch die Produktion der o.g. Antikörper stimuliert, und dass diese dann die Wirkung der NK-Zellen aufheben. Der Regelkreis der Immunantwort ist dann entscheidend gestört, und es kann sich eine Sepsis entwickeln.

Der Nachweis von natürlich vorkommenden anti-AG_{M1}-Antikörpern und damit kreuzreagierenden anti-Gangliosid-Antikörpern, z.B. anti-G_{M1}-Antikörpern, und die erhöhten Spiegel derartiger Antikörper in Seren von Sepsispatienten, bedeutet somit, dass derartige Antikörper eine bisher unberücksichtigte Einflußgröße auf die infektions- bzw. entzündungsspezifische Cytokinkaskade darstellen können, indem sie in den natürlichen Cytokin-Regelkreis eingreifen und diesen durch Störung oder Ausschaltung der NK-Zellen veranlassen können, zu entgleisen und bei dem Patienten zu einer septischen Reaktion auszulösen.

Es ist also davon auszugehen, dass die in allen Sepsisseren, die im Rahmen der o.g. Bestimmungen vermessen wurden, signifikant erhöht gefundenen anti-AG_{M1}- bzw. anti-G_{M1}-Antikörpertiter eine der Voraussetzungen des Entstehens einer Sepsis sind und sich die Anwesenheit derartiger Antikörper sepsisauslösend bzw. sepsisverstärkend auswirkt.

Die Bestimmung derartiger Antikörper eignet sich somit zur Ermittlung der Gefährdungslage (individuellen Disposition) und für die Prognose bei einem Patienten, der als Sepsisrisikopatient einzustufen ist.

Daraus leitet sich ein Verfahren ab, das darin besteht, dass man in einer biologischen Flüssigkeit eines Sepsisrisikopatienten die Anwesenheit und/oder die Menge von anti-AG_{M1}-Antikörpern und damit kreuzreagierenden Antikörpern bestimmt, und im Fall ihres Nachweises sepsisverhütende Maßnahme ergreift. So kann man zu i.S. einer schnellen präventiven therapeutischen Intervention z.B. eine präventive Antibiotikabehandlung durchführen.

Eine Verabreichung geeigneter Substanzen zu den genannten Zwecken ist auch als reine routinemäßige Präventivmaßnahme durchführbar, d.h. ohne vorherige Bestimmung der Antikörper. Das ist jedoch angesichts der Ernsts eines falsch eingeschätzten Sepsisrisikos wenig empfehlenswert ist.

Da ohne äußeren Stress die Antikörpertiter u.U. sehr niedrig sein können, liegt es im Rahmen der Erfindung, die Antikörperbestimmung nach einer *in vivo* Stimulierung der Antikörperbildung eines Sepsisrisikko-Patienten, z.B. vor einem chirurgischen Eingriff, unter Verwendung unbedenklicher Stimulantien zu vorzunehmen. Angesichts der deutlich erhöht gefundenen IgA-Antikörper (vgl. Figuren 2 und 4) soll die Antikörperbestimmung ausdrücklich auch mit geeigneten Assays in Körpersekreten (z.B. Speichel, Schleim) erfolgen können.

Den bei Sepsispatienten erhöhten Titern von anti-Gangliosid(Auto-)Antikörpern kommt somit eine gravierende medizinische Bedeutung zu, nämlich als pathogener Faktor. Mit dieser Erkenntnis lassen sich neue, vielversprechende therapeutische Ansätze entwickeln:
1. Maßnahmen zum intrakorporalen Blockieren der pathogenen Antikörper dadurch, dass man dem Patienten Mittel (die selbstverständlich die zu fordernde Verträglichkeit aufweisen müssen) verabreicht, die geeignet sind, die an AG_{M1} bindenden Antikörper abzusättigen ("zu blockieren").
   Die Ganglioside selbst sind derartige Mittel, und ihre gute Verträglichkeit für humane Patienten ist aus anderen Zusammenhängen, in denen sie bereits verabreicht werden (sie werden in hohen Mengen an Parkinson-Patienten verabreicht), bekannt. Soweit derartige Ganglioside bereits in relevanten Zusammenhängen zu therapeutischen Zwecken an Menschen verabreicht worden sein sollten, ohne dass die o.g. Zusammenhänge erkannt waren, sind die dabei verwendeten Mittel aus dem Schutzbereich der vorliegenden Erfindung auszunehmen.
   Neben der Verabreichung der Ganglioside selbst, in für den erfindungsgemäßen Zweck geeigneten Zubereitungsformen und Mengen, können, wie oben bereits erläutert, auch Ganglioside "simulierende" Substanzen verabreicht werden. Eine Möglichkeit zur Identifizierung derartiger Substanzen, insbesondere solcher, die fester an die Antikörper binden als die Ganglioside selbst, ist weiter oben, im Zusammenhang mit der Definition von "Simulation", skizziert.
   Kandidaten für derartige Substanzen können beispielsweise sein Oligosaccharide, die aus dem Zucker-Tetramer des AG_{M1}-Moleküls - ohne Ceramid- und Sialinsäurerest - bestehen oder diesen enthalten sowie Derivate davon.
2. Entferung der Antikörper aus der Zirkulation des Patienten. Das kann z.B. auf dem Wege einer extrakorporalen Bindung an feste Affinitätsmaterialien ("Plasmapherese") erfolgen. Für die Herstellung der als Binder eingesetzten Plasmapherese-Affinitätssäulen können ggf. auch hochaffine Kohlenhydratstrukturen genutzt werden, die die Antikörper mit höherer Affinität binden als die Ganglioside selbst und die man aufgrund ihrer Toxineigenschaften nicht an eine Patienten verabreichen kann. Zur Frage der Erzeugung einer T-Zell-Anergie kann ergänzend auch noch auf den Inhalt der EP 705 107 A1 und die darin genannten weiteren Fundstellen verwiesen werden.
3. Abschwächung der körpereigenen Produktion der speziellen Antikörper durch Verabreichung von Mitteln für die Blockierung antigenpräsentierender Zellen oder zur Erzeugung einer T-Zellanergie. Diese Maßnahmen können Begleiteffekte der unter 1. genannten Maßnahmen sein, können aber vorteilhaft auch gezielt angewandt werden, indem man andere Verabreichungswege (z.B. oral) wählt bzw. Mengen, die für die gewünschte Abschwächung der Antikörperproduktion geeignet sind ohne für eine Blockierung aller in der Zirkulation eines Sepsisrisikopatienten oder Sepsispatienten vorhandenen Antikörper auszureichen.

## Patentansprüche

1. Verwendung der Ganglioside Asialo-G_{M1} (AG_{M1}) und/oder G_{M1} sowie von Substanzen, die den Kohlenhydratteil dieser Ganglioside im Hinblick auf eine Bindung an anti-AG_{M1}-Antikörper und/oder anti-G_{M1}-Antikörper simulieren, zur Herstellung eines Mittels zur Bindung oder Blockierung von anti-AG_{M1}-Antikörpern und/oder anti-G_{M1}-Antikörpernl die an natürliche Killerzellen (NKC) binden, für die Verabreichung an Sepsisrisikopatienten und Sepsispatienten, bei denen derartige Antikörper nachgewiesen wurden, zur Prävention, Hemmung und Therapie von Sepsis.

2. Verwendung der Ganglioside AG_{M1} und/oder G_{M1} sowie von Substanzen, die den Kohlenhydratteil dieser Ganglioside im Hinblick auf eine Bindung an anti-AG_{M1}-Antikörper und/oder anti-G_{M1}-Antikörper simulieren, zur Herstellung von Mitteln zur Blockierung antigenpräsentierender Zellen oder zur Erzeugung einer T-Zellanergie bei Sepsisrisikopatienten.

3. Verwendung der Ganglioside AG_{M1} und/oder G_{M1} sowie von Substanzen, die den Kohlenhydratteil dieser Ganglioside im Hinblick auf eine Bindung an anti-G_{M1}-Antikörper und/oder anti-AG_{M1}-Antikörper simulieren, zur Herstellung eines Affinitätsmaterials zur extrakorporalen Entfernung von anti-AG_{M1}-Antikörpern und/oder anti-G_{M1}-Antikörpern, die an natürliche Killerzellen binden, aus dem Blutkreislauf eines Sepsisrisikopatienten oder Sepsispatienten.

4. Verwendung nach einem der Ansprüche 1 bis 3, **dadurch gekennzeichnet, dass** die Substanzen, die den Kohlenhydratteil dieser Ganglioside im Hinblick auf eine Bindung an anti-AG_{M1}-Antikörper und/oder anti-G_{M1}-Antikörper simulieren, **dadurch gekennzeichnet sind, dass** sie mit Hilfe eines Screeningverfahrens zu erkennen sind, bei dem man ein Serum mit einem hohen Gangliosid-Antikörper-Titer mit einer zu prüfenden Substanz versetzt, danach das Bindungsverhalten der Antikörper aus der Probe an ihren spezifischen Bindungspartner bestimmt und ein gegenüber der substanzfreien Probe vermindertes Bindungsverhalten mit einer Gangliosidsimulation korreliert.

5. Verwendung nach Anspruch 2 zur Herstellung eines Mittels für die Blockierung antigenpräsentierender Zellen oder zur Erzeugung einer T-Zellanergie für die Verabreichung durch Injektion oder für die orale Verabreichung.

6. Mittel zur Prävention und Behandlung von Sepsis, das neben pharmazeutisch annehmbaren Trägern Wirkbestandteile enthält, die an AG_{M1}- und/oder G_{M1}-Antikörper auf eine Weise binden, dass die Bindung dieser Antikörper an natürliche Killerzellen (NKC) ganz oder teilweise verhindert wird.
